(19) Europäisches Patentamt
European Patent Office
Office européen des brevets

(11) **EP 3 327 599 A1**

(12) **EUROPEAN PATENT APPLICATION**
published in accordance with Art. 153(4) EPC

(43) Date of publication:
**30.05.2018 Bulletin 2018/22**

(51) Int Cl.:
***G06F 19/00*** (2018.01)

(21) Application number: **16760782.9**

(86) International application number:
**PCT/IB2016/054324**

(22) Date of filing: **20.07.2016**

(87) International publication number:
**WO 2017/013604 (26.01.2017 Gazette 2017/04)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **20.07.2015 PT 2015108706**

(71) Applicant: **Universidade de Trás-os-Montes e Alto Douro**
**5000-911 Vila Real (PT)**

(72) Inventors:
• **BEZERRA, Rui Manuel Furtado**
**5000-801 Vila Real (PT)**
• **DIAS, José Albino Gomes Alves**
**5000-801 Vila Real (PT)**

(74) Representative: **Ferreira, Maria Silvina Clarke, Modet & Co**
**Av. Casal Ribeiro, N°50-3° andar**
**1000-93 Lisboa (PT)**

(54) **METHOD FOR DETERMINING ENZYME INHIBITION KINETICS IN THE SIMULTANEOUS PRESENCE OF TWO INHIBITORS WHEN ONE OF THE INHIBITORS IS A REACTION PRODUCT**

(57)    This application refers to a method for the determination of the inhibition kinetic of enzymes, considering the simultaneous presence of two inhibitors when one of them is a product of the reaction, which method can be applied to different existing models of kinetics, and based on Michaelis-Menten integrated equations.

**Description**

**Technical Domain**

This application is related to a method for the determination of the inhibition kinetics of enzymes, considering the simultaneous presence of two inhibitors, where one of them is a product of the reaction, which method can be applied to different models of existing kinetics, and based on Michaelis-Menten,s integrated equations.

**Background of the Invention**

Several authors have reported the possibility of studying the enzymatic kinetics in presence of two inhibitors, by taking into account the integrated equation of Michaelis-Menten. However, the practical demonstration of how this is achieved is not mentioned.

Any experimental study on kinetics of an enzymes catalyzed reaction, does display a fundamental incompatibility between data and the underlying model. The enzymatic kinetic of an enzyme is generally evaluated by analyzing the reaction speeds, but experimental measurements do not determine directly the speeds. In order to avoid this incompatibility, the model must be integrated to give a description of the reaction course, or the data must be differentiated (starting speeds determination). However, there is no precise procedures do estimate starting speeds, and in most cases this is carried out drawing the tangent to the experimental curve of the product in function of time. This procedure is theoretically incorrect, as it doesn't assume a kinetic model, and experimental data can be already influenced, for example by inhibition of the product.

The Michaels-Menten integrated equation has the advantage that the initial addition of an inhibitor product can be eliminated, and the inhibition constant ($K_i$) can be determined under conditions which do not require the separate addition of the reaction products, what allows for example determining more precisely the inhibition constant of a particular isomer of the reaction product, once that it is based on the correct and complete stereo chemical purity. However, it can also be chosen to add the initial inhibitor product (Bezerra et al., 2013). The integrated equation has another great advantage because it allows studying the inhibition of a second inhibitor that is not product of the reaction, without neglecting the inhibition of the reaction product.

It's not of our knowledge the application of Michaelis-Menten integrated equations in presence of two inhibitors in reaction medium. However, the integrated equations are of particular interest in studies with two different inhibitors, once that product inhibition is frequently found as a regulation mechanism of many enzymes. Thus, to study inhibition kinetics of this kind of enzymes, we need to consider the simultaneous presence of two inhibitors as one of them is reaction product, and it is always present.

The main objective of the invention of this application is to develop and apply integrated equations for kinetic analysis of reactions carried out in presence of two inhibitors when one of them is reaction product. Must be pointed out that with the Michaelis-Menten speed equations it is not possible to determine kinetic parameter with the simultaneous presence of two inhibitors, once that constants are associated. Only when a Michaelis-Menten equation is integrated the parameter cease to be associated, as one of the inhibitors is always increasing, and the other remains constant during the reaction.

One of the main warnings regarding the use of the integrated Michaelis-Menten equation is the possibility of gradual enzymatic activity loss under the conditions of essay, along a great part of the product time curve. Nevertheless, nothing prevents the use of the integrated equation, considering only the experimental points commonly used to determine initial rates (when consumed substrate is less than about 10%). Thus, in the example shown below, the integrated equations are applied only to experimental points commonly used to determine initial rates.

The kinetic study of the inhibition of enzymes considering simultaneous presence of two inhibitors, when one of them is reaction product, is only possible taking into account the Michaelis-Menten integrated equation. To date, the inventors did not notice any work disclosure that allows to establish, based on experimental results, the kinetic parameter of two simultaneous inhibitors being one of them reaction product.

Currently, the determination of kinetic constants only considers one inhibitor, when enzymes are usually inhibited by various products, including the reaction product. The development of inhibitors comes across the difficulty of studying the effect of this inhibitor without neglecting the inhibition of reaction product.

International application WO 03/106698 A1 refers to a method working with rate equations (velocity versus substrate concentration). While present patent application works with integrated equations (Table 2) (values product versus time). WO 03/106698 A1 works only with an inhibitor at a time, while the method of present patent application allows the simultaneous study of two inhibitors. Must be pointed out that the determination of inhibition constants, $K_{ic1}$ $K_{iu1}$, by the Michaelis-Menten equation (initial rates), requires initially adopting phosphate, in another set of tests without urea, which does not happen with the proposed methodology of the present invention.

The development of new drugs requests the study of new inhibitors, wherein in cases where the product is the reaction inhibitor we face the problem of being present a reaction with two inhibitors. What is done in such cases is to neglect reaction product as an inhibitor, studying it when the time tends to zero. This is a practical way to restrict inhibition effect but not cancel it. The methodology proposed here, unlike the usual process, is theoretically correct and studies simultaneously the inhibition of both inhibitors allowing determine kinetic constants for the two inhibitors. The method has further the advantage of allowing reaction with only a substrate concentration, and one for the second inhibitor, thus saving reagents and time spent.

**Summary of the Invention**

The present invention refers to a method for the determination of the enzymatic inhibition kinetics in simultaneous presence of two inhibitors, where one of the inhibitors is reaction product, which comprises the following steps:

- reacting a substrate and an enzyme in the presence of two inhibitors, where one inhibitor is the reaction product;
- monitoring the concentrations of the product formed, the remaining substrate and inhibitor 1, reaction product, reaction at various time intervals, through a methodology adequate to determine substrate and product;
- determining reaction time needed to obtain a particular reaction product concentration through the integrated Michaelis-Menten equation of Table 2;
- determining the difference between reaction real time, and the time obtained by the integrated Michaelis-Menten equation of Table 2;
- calculating the SSE values using the values determined in the previous step;
- performing a non-linear regression analysis to determine the values of the kinetic parameters that minimizes the SSE values for each inhibition model;
- selecting adequate inhibition model, by comparing minimized value SSE and Akaike's information criterion; and
- determining kinetic parameters for selected inhibition model, through the application of Michaelis-Menten integrated equation corresponding to selected model.

In one embodiment, the adequate methodology to determine the substrate, and the reaction product comprises the absorbency readings, or the use of another analytical method as, for example mass spectrophotometry, polarography and refraction index.

In another embodiment the kinetic parameters determined for the selected inhibition model comprise: *Km, Kic1, Kiu1, Kic2, Kiu2 and Vmax.*

**General Description of the Invention**

Present application arises from the need to determine the kinetic inhibition, when there is the simultaneous presence of two inhibitors, and when one of them is reaction product.

The proposed method allows discriminating the type of inhibition and kinetic constants as a function of product concentration and reaction time, considering the simultaneous presence of two inhibitors, when one of them is reaction product.

The object is to determine kinetic enzymatic models and kinetic parameters applying to both inhibitors simultaneously present in the reaction medium, one of them being reaction product.

The herein described method can be used to develop software allowing, when in connection with a spectrophotometer or other detection device, determining on line the kinetic model in question, and the kinetic parameter values.

Thus, the present invention is based on the use of the Michaelis-Menten integrated equation, considering that there is an inhibitor remaining constant, and another one that increases its concentration over time. This makes the inhibition constants not associated.

In order to determine the initial rates of enzyme catalyzed reactions without theoretical mistakes, it is necessary to consider the use of the Michaelis-Menten integrated equation. When the reaction product is an inhibitor, that approach is particularly important. However, kinetic studies generally involve other inhibitors evaluation in addition to reaction product. The occurrence of such situations emphasizes the importance of using the Michaelis-Menten equation, assuming that more than one reaction inhibitor is simultaneously present, as the product is always present. This methodology is illustrated in the example below, by the reaction catalyzed by alkaline phosphatase inhibited by phosphate (reaction product, inhibitor 1), and urea (second inhibitor, inhibitor 2).

The method of present invention allows the study of enzymatic kinetics with two inhibitors without despising the reaction product inhibition. Kinetic determinations will not be correct without attention to inhibition of the product. Thus, the herein proposed method solves a technical problem in this area, which until now has been neglected, that is the effect of product inhibition.

**Detailed Description of the Invention**

All common types of linear inhibitions are included in the linear mixed inhibition model (MI), once that some inhibition constants can tend to infinite, meaning that they are irrelevant, giving rise to other linear models. Assuming a mixed linear inhibition (MI), the following Michaelis-Menten rate equation is obtained:

$$v = \frac{V_{\max}[S_0]}{K_m\left(1 + \frac{[I]}{K_{ic}}\right) + [S]\left(1 + \frac{[I]}{K_{iu}}\right)} \quad (eq.\,1)$$

Where:

$K_m$ is a Michaelis constant;
$K_{ic}$ is dissociation constant of the enzyme-inhibitor complex;
$K_{iu}$ is dissociation constant of the enzyme-substrate-inhibitor complex;
Vmax is inhibition maximal rate;
V is reaction initial rate;
[I] is inhibitor concentration
$[S_0]$ is substrate initial concentration.

Given the presence of two different inhibitors $I_1$ e $I_2$, as well as different inhibition constants $K_{ic1}$ e $K_{ic2}$ ou $K_{iu1}$ e $K_{iu2}$ the following rates equation is obtained:

$$v = \frac{V_{\max}[S_0]}{K_m\left(1 + \frac{[I_1]}{K_{ic1}} + \frac{[I_2]}{K_{ic2}}\right) + [S]\left(1 + \frac{[I_1]}{K_{iu1}} + \frac{[I_2]}{K_{iu2}}\right)} \quad (eq.\,2)$$

Assuming that $I_1$ inhibitor is also formed during the reaction:

$$[I_1] \ = \ [Pt] \ + \ [I_{1,0}],$$

$$[S_0] \ - \ [St] \ = \ [Pt],$$

[St] is substrate concentration during time t,
Subscript "0", for example, So, $I_{1,0}$, meaning an initial concentration of $I_1$ at t = 0.
Thus, the obtained rates equation is:

$$v = -\frac{d[S]}{dt} = \frac{V_{\max}[S_0]}{K_m\left(1 + \frac{[I_{1,0}]}{K_{ic1}} + \frac{[P_t]}{K_{ic1}} + \frac{[I_2]}{K_{ic2}}\right) + [S]\left(1 + \frac{[I_{1,0}]}{K_{iu1}} + \frac{[P_t]}{K_{iu1}} + \frac{[I_2]}{K_{iu2}}\right)} \quad (eq.\,3)$$

In equation 3, the inhibition constants $K_{ic1}$ e $K_{ic2}$ ou $K_{iu1}$ e $K_{iu2}$ are associated, however, after integration assuming that Pt increases with time, the inhibition constants cease being associated.
Assuming that Vmax = [E]kv where [E] is the enzyme concentration, and kv a rate constant, and integrating from zero to time t, and from [So] to [St] is obtained that:

$$t = -\left(-\frac{1}{[E]k_v}\right)\left(K_m\left(1 + \frac{[S_0]}{Kic_1} + \frac{[I_{1,0}]}{Kic_1} + \frac{[I_2]}{Kic_2}\right)\ln\frac{[S_t]}{[S_0]}\right.$$

$$+ \left(1 - \frac{K_m}{Kiu_1} + \frac{[S_0]}{Kiu_1} + \frac{[I_{1,0}]}{Kiu_1} + \frac{[I_2]}{Kiu_2}\right)([S_t] - [S_0])$$

$$\left.+ \frac{1}{2}\left(-\frac{1}{Kiu_1}\right)([S_t]^2 - [S_0]^2)\right) \quad (eq.\,4)$$

The anterior equation 4 can be simplified for the simplest models of inhibition (CI - competitive, NCI - noncompetitive, UCI - without competitiveness, and finally WI - considering no inhibition). These models are simplifications of MI model, assuming that different constants ($K_{iu1}$, $K_{ic1}$, $K_{iu2}$, $K_{ic2}$) tend to infinity.

The model WI without inhibition can be obtained by simplification of MI model, assuming Kic e $K_{iu}$, both for inhibitor 1, and for inhibitor 2, as well as for infinity ($\infty$). So it is also possible to obtain models CI of competitive inhibition, considering $K_{iu1} = \infty$ e $K_{iu2} = \infty$; NCI noncompetitive, considering $k_{iu1} = kici$ and $k_{iu2} = k_{ic2}$; UI without competitiveness, considering $K_{ic1} = \infty$ and $K_{ic2} = \infty$.

In addition to the models above, present invention method can be applied to any existing kinetics model, and to any rate equation.

Those equations are always equations to foresee reaction time necessary for obtaining a determined product quantity (implicit form).

Present invention method for the determination of enzymatic inhibition kinetics, comprises two steps:

  1) a discrimination between available models; and
  2) a parameters estimate.

Models of competitive inhibition (CI), noncompetitive inhibition (NCI), and inhibition without competition (UCI), have the same number of parameters and therefore it is sufficient to compare the SSE value to discriminate. Among the methods to compare and discriminate models the information criterion from Akaike (AIC) was chosen, see Akaike, H. 1974. Being both inhibitors of same type, for example, competitive, the model to be applied is of the competitive type. In this case, a discrimination of kinetic models was ended and the parameters could be obtained from the discriminated model. In the cases where the two models are not of same type, the discriminated inhibition will be the mixed one. Then one can study only the inhibitor, the reaction product, and then the other inhibitor, by fixing the constants for first inhibitor, as explained in the example presented below.

**Example**

Material and Methods

The reagents are available from large suppliers such as Merck. Disodium salt solutions of 4-nitro phenyl phosphate were prepared in 20 mM concentrations. Reactions were performed at 37°C containing 9.5 $\mu$g enzyme in 2.75 ml reaction volume, and different amounts of 4-nitrophenylphosphate (between 18.4 and 1500.0 $\mu$M Tris / HCl buffer 0.1 M, pH 9.0. The product amount (4-nitro phenol) formed in several time intervals (10 to 60 s) was determined by absorbency at 405 nm, using a spectrophotometer, under conditions not surpassing 10% of substrate depletion. Another similar set of reaction experiments was also done in presence of 1.25 M of urea (inhibitor).

Results and Discussion

Resource was made to the use of Microsoft Office Excel software and the supplement Solver to determine kinetic parameters from Michaelis-Menten equation.

Thus the following experimental data have been inserted (Table 1), and the following calculations done with Microsoft Office Excel software:

-    Data from the reaction product were inserted into column B (Table 1) ;

- Data from the reaction time were inserted at column C (Table 2);

- Remaining substrate in each test was calculated into column K;

- Integrated equations (Table 2), which allow determining the reaction time necessary to obtain the reaction product experimentally achieved, were inserted into column D;

- Concentration of substrate, concentration of inhibitor 1 (reaction product), concentration of inhibitor 2 (in this case an inhibitor that is not reaction product, for example urea), and the protein concentration in the enzymatic test, were inserted into columns E, F, G e H, respectively;

- The square of the difference between the actual time (column C) and the time obtained by the integrated equation (column D) was calculated into column I;

- Values of column I were used to calculate the sum of squared deviations (SSE), used in kinetic parameters optimization by nonlinear regression (kinetic parameters minimizing the sum of square divisions).

Table 1 – Values of product concentration, of time and of substrate concentration to be inserted in the worksheets of Excel, where the values till line 75 refer to essays without urea, and the other values have been tested with ureia 1,25 mM

| Line Excel | Product (µM) Column B | Time (s) Column C | Substrate (µM) Column E |
|---|---|---|---|
| 10 | 0.000 | 0 | 18.37 |
| 11 | 0.480 | 10 | 18.37 |
| 12 | 0.933 | 20 | 18.37 |
| 13 | 1.350 | 30 | 18.37 |
| 14 | 1.814 | 40 | 18.37 |
| 15 | 2.209 | 50 | 18.37 |
| 16 | 2.646 | 60 | 18.37 |
| 17 | | | |
| 18 | 0.000 | 0 | 29.57 |
| 19 | 0.734 | 10 | 29.57 |
| 20 | 1.377 | 20 | 29.57 |
| 21 | 1.958 | 30 | 29.57 |
| 22 | 2.621 | 40 | 29.57 |
| 23 | 3.200 | 50 | 29.57 |
| 24 | 3.687 | 60 | 29.57 |
| 25 | | | |
| 26 | 0.000 | 0 | 39.79 |
| 27 | 0.765 | 10 | 39.79 |
| 28 | 1.485 | 20 | 39.79 |

| Line Excel | Product (µM) Column B | Time (s) Column C | Substrate (µM) Column E |
|---|---|---|---|
| 58 | 0.000 | 0 | 750 |
| 59 | 1.332 | 10 | 750 |
| 60 | 2.860 | 20 | 750 |
| 61 | 4.369 | 30 | 750 |
| 62 | 6.324 | 40 | 750 |
| 63 | 7.974 | 50 | 750 |
| 64 | 9.379 | 60 | 750 |
| 65 | | | |
| 66 | 0.000 | 0 | 1500 |
| 67 | 1.762 | 10 | 1500 |
| 68 | 3.379 | 20 | 1500 |
| 69 | 4.949 | 30 | 1500 |
| 70 | 6.821 | 40 | 1500 |
| 71 | 8.461 | 50 | 1500 |
| 72 | 10.120 | 60 | 1500 |
| 73 | | | |
| 74 | 0.000 | 0 | 18.37 |
| 75 | 0.468 | 10 | 18.37 |
| 76 | 0.815 | 20 | 18.37 |

| Line Excel | Product (µM) Column B | Time (s) Column C | Substrate (µM) Column E |
|---|---|---|---|
| 106 | 0.000 | 0 | 90 |
| 107 | 0.733 | 10 | 90 |
| 108 | 1.492 | 20 | 90 |
| 109 | 2.209 | 30 | 90 |
| 110 | 2.921 | 40 | 90 |
| 111 | 3.604 | 50 | 90 |
| 112 | 4.310 | 60 | 90 |
| 113 | | | |
| 114 | 0.000 | 0 | 200 |
| 115 | 0.965 | 10 | 200 |
| 116 | 2.025 | 20 | 200 |
| 117 | 2.969 | 30 | 200 |
| 118 | 3.880 | 40 | 200 |
| 119 | 4.879 | 50 | 200 |
| 120 | 5.860 | 60 | 200 |
| 121 | | | |
| 122 | 0.000 | 0 | 750 |
| 123 | 0.922 | 10 | 750 |
| 124 | 2.072 | 20 | 750 |

Table 1 - Continuation

| Line Excel | Product (µM) Column B | Time (s) Column C | Substrate (µM) Column E | Line Excel | Product (µM) Column B | Time (s) Column C | Substrate (µM) Column E | Excel | Product (µM) Column B | Time (s) Column C | Substrate (µM) Column E |
|---|---|---|---|---|---|---|---|---|---|---|---|
| 29 | 2.327 | 30 | 39.79 | 77 | 1.092 | 30 | 18.37 | 125 | 3.326 | 30 | 750 |
| 30 | 2.903 | 40 | 39.79 | 78 | 1.422 | 40 | 18.37 | 126 | 4.317 | 40 | 750 |
| 31 | 3.568 | 50 | 39.79 | 79 | 1.705 | 50 | 18.37 | 127 | 5.470 | 50 | 750 |
| 32 | 4.292 | 60 | 39.79 | 80 | 2.005 | 60 | 18.37 | 128 | 6.730 | 60 | 750 |
| 33 | | | | 81 | | | | 129 | | | |
| 34 | 0.000 | 0 | 48.87 | 82 | 0.000 | 0 | 29.57 | 130 | 0.000 | 0 | 1500 |
| 35 | 0.708 | 10 | 48.87 | 83 | 0.609 | 10 | 29.57 | 131 | 1.331 | 10 | 1500 |
| 36 | 1.543 | 20 | 48.87 | 84 | 1.002 | 20 | 29.57 | 132 | 2.561 | 20 | 1500 |
| 37 | 2.400 | 30 | 48.87 | 85 | 1.395 | 30 | 29.57 | 133 | 3.758 | 30 | 1500 |
| 38 | 3.179 | 40 | 48.87 | 86 | 1.821 | 40 | 29.57 | 134 | 5.055 | 40 | 1500 |
| 39 | 3.962 | 50 | 48.87 | 87 | 2.234 | 50 | 29.57 | 135 | 6.349 | 50 | 1500 |
| 40 | 4.662 | 60 | 48.87 | 88 | 2.601 | 60 | 29.57 | 136 | 7.650 | 60 | 1500 |

Table 2 - Integred equations obtained through present invention for the different models of Michaelis-Menten considering different types of inhibition

| Model | Michaelis-Menten Equiation | Integrated Equations | Excel Equations * (Cell D10) |
|---|---|---|---|
| WI | $v_o = \dfrac{V_{max}[S]}{K_m+[S]}$ | $S_t$<br>$\alpha = K_m \ln \dfrac{[S_t]}{[S_0]}$<br>$\beta = ([S_t] - [S_0])$<br>$t = -\left(-\dfrac{1}{[E]k_v}\right)(\alpha + \beta)$ | K10 =E10-(B10-F10)<br>L10 =$B$1*LN(K10/E10)<br>M10 = (K10-E10)<br>Tempo calculado =-1/(H10*$B$2)*(L10+M10)<br>Assumir: $K_m$=$B$1, kv=$B$2 |
| CI | $v_o = \dfrac{V_{max}[S]}{K_m\left(1+\frac{[I]}{K_{ic}}\right)+[S]}$ | $S_t$<br>$\alpha = K_m\left(1+\dfrac{[S_0]}{Kic_1}+\dfrac{[I_1]}{Kic_1}+\dfrac{[I_2]}{Kic_2}\right)\ln\dfrac{[S_t]}{[S_0]}$<br>$\beta = \left(1-\dfrac{K_m}{Kic_1}\right)([S_t]-[S_0])$<br>$t = -\left(-\dfrac{1}{[E]k_v}\right)(\alpha+\beta)$ | K10 =E10-(B10-F10)<br>L10 = $B$1*(E10/$B$2+F10/$B$2+G10/$B$3+1)*LN(K10/E10)<br>M10 = (1-$B$1/$B$2)*(K10-E10)<br>Tempo calculado =(-1/(H10*$B$4))*(L10+M10)<br>Assumir: $K_m$=$B$1, Kic1=$B$2, Kic2=$B$3, kv=$B$4 |
| NCI | $v_o=\dfrac{V_{max}[S]}{K_m\left(1+\frac{[I]}{K_{ic}}\right)+[S]\left(1+\frac{[I]}{K_{iu}}\right)}$<br>$(K_{ic}=K_{iu})$ | $S_t$<br>$\alpha = K_m\left(1+\dfrac{[S_0]}{Kic_1}+\dfrac{[I_1]}{Kic_1}+\dfrac{[I_2]}{Kic_2}\right)\ln\dfrac{[S_t]}{[S_0]}$<br>$\beta = \left(1-\dfrac{K_m}{Kic_1}+\dfrac{[S_0]}{Kiu_1}+\dfrac{[I_1]}{Kiu_1}+\dfrac{[I_2]}{Kiu_2}\right)([S_t]-[S_0])$<br>$\gamma = \dfrac{1}{2}\left(-\dfrac{1}{Kiu_1}\right)([S_t]^2-[S_0]^2)$<br>$t = -\left(-\dfrac{1}{[E]k_v}\right)(\alpha+\beta+\gamma)$ | K10= E10-(B10-F10)<br>L10= $B$1*(E10/$B$2+F10/$B$2+G10/$B$3+1)*LN(K10/E10)<br>M10= (1-$B$1/$B$2+E10/$B$2+F10/$B$2+G10/$B$3)*(K10-E10)<br>N10= -1/(2*$B$2)*(K10^2-E10^2)<br>Tempo calculado = (-1/(H10*$B$4))*(L10+M10+N10)<br>Assumir: $K_m$=$B$1, kiu1= kic1=$B$2, kiu2 = kic2=$B$3, kv=$B$4 |

| | | | |
|---|---|---|---|
| UCI | $v_o = \frac{V_{max}[S]}{K_m + [S]\left(1 + \frac{[I]}{K_{iu}}\right)}$ | $S_t$<br>$\alpha = K_m \ln\frac{[S_t]}{[S_0]}$<br>$\beta = \left(1 + \frac{[S_0]}{Kiu_1} + \frac{[I_1]}{Kiu_1} + \frac{[I_2]}{Kiu_2}\right)([S_t] - [S_0])$<br>$\gamma = \frac{1}{2}\left(-\frac{1}{Kiu_1}\right)([S_t]^2 - [S_0]^2)$<br>$t = -\left(-\frac{1}{[E]k_v}\right)(\alpha + \beta + \gamma)$ | K10= E10-(B10-F10)<br>L10= $B$1*LN(K10/E10)<br>M10= (1+E10/$B$2+F10/$B$2+G10/$B$3)*(K10-E10)<br>N10= -1/(2*$B$2)*(K10^2-E10^2)<br>Tempo calculado = (-1/(H10*$B$4))*(L10+M10+N10)<br>Assumir: Km=$B$1, Kiu1=$B$2, Kiu2=$B$3, kv=$B$4 |
| MI | $v_o = \frac{V_{max}[S]}{K_m\left(1 + \frac{[I]}{K_{ic}}\right) + [S]\left(1 + \frac{[I]}{K_{iu}}\right)}$ | $S_t$<br>$\alpha = K_m\left(1 + \frac{[S_0]}{Kic_1} + \frac{[I_1]}{Kic_1} + \frac{[I_2]}{Kic_2}\right)\ln\frac{[S_t]}{[S_0]}$<br>$\beta = \left(1 - \frac{K_m}{Kic_1} + \frac{[S_0]}{Kiu_1} + \frac{[I_1]}{Kiu_1} + \frac{[I_2]}{Kiu_2}\right)([S_t] - [S_0])$<br>$\gamma = \frac{1}{2}\left(-\frac{1}{Kiu_1}\right)([S_t]^2 - [S_0]^2)$<br>$t = -\left(-\frac{1}{[E]k_v}\right)(\alpha + \beta + \gamma)$ | K10= E10-(B10-F10)<br>L10= $B$1*(E10/$B$2+F10/$B$2+G10/$B$3+1)*LN(K10/E10)<br>M10= (1-$B$1/$B$2+E10/$B$5+F10/$B$5+G10/$B$6)*(K10-E10)<br>N10= -1/(2*$B$5)*(K10^2-E10^2)<br>Tempo calculado = (-1/(H10*$B$4))*(L10+M10+N10)<br>Assumir: Km=$B$1, Kic1=$B$2, Kic2=$B$3, kv=$B$4, Kiu1=$B$5, Kiu2=$B$6 |

*Legenda: WI-Sem Inibição, CI- Inibição Competitiva, NCI- Inibição não Competitiva, UCI- Inibição Acompetitiva, MI- Inibição Mista

**[0037]** As stated before, present invention method for the determination of enzymatic inhibition kinetics, comprises two steps:

- a discrimination between available models (Tables 3 and 4); and
- Parameters estimate (Table 5).

As per Table 3, the CI, NCI e UCI models have the same number of parameters and so it is enough to compare the SSE value to discriminate the better model.

Comparison reveals that NCI is better than any other model, once that SSE value is lesser.

However, the lesser value of SSE was obtained with MI model.

However, this model has a larger number of parameters which precludes a direct comparison with simpler models.

So, a statistical discrimination is necessary to compare simpler models with MI model (more complex).

Among the methods to compare and discriminate models the information criterion from Akaike (AIC) was chosen, (Table 4) .

Results show that the better model is MI model, with a probability of about 100%, and must be preferred.

Vmax ($\mu$mol.min-1.mg-1) can be obtained from ($\mu$M.s$^{-1}$. ($\mu$g/2,75 ml)$^{-1}$).

Table 3 - Values of the sum of squared errors (SSE) for the different models.

|  | wi | CI | NCI | UCI | MI |
|---|---|---|---|---|---|
| **SSE** | 3976.3 | 1455.2 | 468.7 | 1019.0 | 385.1 |
| **p** | 2 | 4 | 4 | 4 | 6 |
| **n** | 112 | 112 | 112 | 112 | 112 |

*Legend: p- number of model parameters; n- number of test points.*

Table 4 - Summary of discrimination model using Akaike information criterion methodology in the presence of phosphate and urea.

| Model s A/B | $SSE_A$ | $SSE_B$ | n | $P_A$+1 | $P_B$+1 | $AIC_A$ | $AIC_B$ | $AIC_{cA}$ | $AIC_{cB}$ | $\Delta$ | Prob. B |
|---|---|---|---|---|---|---|---|---|---|---|---|
| **WI/MI** | 3976.3 | 385.1 | 112 | 3 | 7 | 405.8 | 152.3 | 406.0 | 153.4 | -252.6 | 1.0 |
| **NCI/M I** | 468.7 | 385.1 | 112 | 5 | 7 | 170.3 | 152.3 | 170.9 | 153.4 | -17.5 | 0.99984 |

*Legend: A/B - Comparison of the two models (A and B) where A is the model with fewer parameters; $P_A$ and $P_B$ - Number of models A and B, parameters respectively; AICc - AIC corrected; $\Delta$ - difference between the AICc values of the two compared models; Prob. B-Probability of B being correct.*

Table 5- Summary of obtained constants with better model (MI Model).

| Michaelis-Menten | Km ($\mu$M) | Kic1 ($\mu$M) | Kiu1 ($\mu$M) | Kic2 (M) | Kiu2 (M) | Vmax ($\mu$mol.min$^{-1}$ .mg$^{-1}$) |
|---|---|---|---|---|---|---|
| **Integrated Equation** | 41.4$\pm$1.2 | 7.0$\pm$1.5 | 3.3x10$^6$$\pm$0.0 | 2.4$\pm$0.3 | 3.3$\pm$0.3 | 3.0$\pm$0.0 |

By analyzing Table 5, it is evident that $K_{iu1}$ (inhibition constant noncompetitive of phosphate) tends to infinity (is to big comparing to $K_{ic1}$) and therefore the phosphate (inhibitor 1) is a predominantly competitive inhibitor. Urea (inhibitor 2) presents similar values for $K_{iu2}$ and $K_{ic2}$, showing a predominance of noncompetitive inhibition. If both inhibitors are of same type, for example, competitive, it will be expected that better model is the competitive type. In this case, a kinetic model discrimination was ended and the parameters could be obtained from the discriminated better model. The results on Table 5 do not fit into this situation, because phosphate has an inhibition behavior predominantly competitive, and urea has an inhibition behavior predominantly noncompetitive (or eventually mixed). Thus, in order to categorically say that phosphate is an inhibitor competitive, it is necessary to analyze the results, considering only the tests with phosphate (present or not from the beginning). This study did not consider the initial presence of phosphate, but only the reaction product and this obviously increases with time. Could have been also used initial concentrations of phosphate (Bezerra et al., 2013). To do so, it is necessary to analyze the test points obtained in the absence of urea. The obtained results of this situation are shown in Tables 6 and 7, and it is possible to conclude that CI is better than MI, with a probability of 77%, and still better then WI, with a probability of 100%. The inhibition constant obtained was $K_{ic1}$ = 10,7 $\mu$M.

**Table 6** - Square sum of errors (SSE) for different models that take only the tests in the absence of urea.

|  | wi | CI | NCI | UCI | MI |
|---|---|---|---|---|---|
| SSE | 1327.5 | 83.1 | 120.2 | 114.8 | 83.1 |
| p | 2 | 3 | 3 | 3 | 4 |
| n | 62 | 62 | 62 | 62 | 62 |

*Legend: p- number of model parameters; n- number of test points.*

**Table 7-** Summary of discrimination of models using Akaike information criterion methodology taking only tests in the absence of urea.

| Models A/B | $SSE_A$ | $SSE_B$ | n | $P_A$+1 | $P_B$+1 | $AIC_A$ | $AIC_B$ | $AIC_{cA}$ | $AIC_{cB}$ | $\Delta$ | Prob B |
|---|---|---|---|---|---|---|---|---|---|---|---|
| WI/CI | 1327.5 | 83.1 | 62 | 3 | 4 | 196.0 | 26.1 | 1964.4 | 26.8 | - 169.6 | 1.00 |
| CI/MI | 83.1 | 83.1 | 62 | 4 | 5 | 26.1 | 28.1 | 26.8 | 29.2 | 2.4 | 0.23 |

*Legend: A/B - Comparison of the two models (A and B) where A is the model with fewer parameters; $P_A$ and $P_B$ - Number of models A and B parameters respectively; AICc - AIC corrected; $\Delta$ - difference between the AICc values of the two compared models; Prob. B- Probability of B being correct.*

With the object of evaluating the pattern of urea inhibition, only an equation MI modified is used. As phosphate is a reaction product, it is not possible to study a urea inhibition in the absence of phosphate. Thus, it is necessary to copy the model of MI equation (Table 2) to five worksheets, and give for example, the following names for the five sheets (WIm, to CIm, NCIm, UCIm e MI; "m" meaning modified). Modifications of model MI are necessaries because phosphate inhibition constants ($K_{ic1}$ and $K_{iu1}$ are now fixed values, and it is not possible make that with the original equations. So, $K_{ic1}$ (cell B2) and $K_{iu1}$ (cell B5) shall be fixed ($K_{ic}1 = 10,7$ uM e $Ki_{u1} = \infty$ (e.g. $3.3x10^6$ uM obtained at Table 5), this is, converting them into non variable parameters. Furthermore, other slight alterations in each of the five worksheets must be executed, as explained at Table 8. Before execution of linear regression it is necessary to remove from the "by change of cell" option (from Solver dialogue box) the cells referring to kinetic constants $K_{ic1}$ and $Kiu1$. The discrimination result at Tables 9 and 10, show that urea is a mixed inhibitor with a probability of 57%. The obtained constants were $Kic2 = 2,6$ M and $Kiu2 = 3.3$ M. Using this methodology it is possible to state that phosphate is a competitive inhibitor, and urea a mixed inhibitor, with the inhibition constants presented at Table 5.

**Table 8-** Modifications in model MI do evaluate the inhibition pattern of second inhibitor (urea) (see text).

| MI Modified Model | Alterations to be made in Model MI | Number of remaining parameters |
|---|---|---|
| WIm | Remove [I2]/Kic2 (G10/$B$3) and [I2]/Kiu2 (G10/$B$6) | 2 |
| CIm | Remove [I2]/Kiu2 (G10/$B$6) | 3 |
| NCIm | Change [I2]/Kiu2 (G10/$B$6) to [I2]/Kic2 (G10/$B$3) | 3 |
| UCIm | Remove [I2]/Kic2 (G10/$B$3) | 3 |
| MI | Don't remove anything | 4 |

**Table 9 -** Square sum of errors (SSE) for different models that take the modifications of Table 8 (in presence of phosphate and urea).

|  | wim | CIm | NCIm | UCIm | MIm |
|---|---|---|---|---|---|
| SSE | 4258.3 | 1456.0 | 402.3 | 1096.9 | 392.6 |
| p | 2 | 3 | 3 | 3 | 4 |

(continued)

|  | wim | CIm | NCIm | UCIm | MIm |
|---|---|---|---|---|---|
| n | 112 | 112 | 112 | 112 | 112 |

*Legend: p- number of model parameters; n- number of test points.*

**Table 10 -** Summary of discrimination model using Akaike information criterion methodology, taking the modifications from Table 8 (in presence of phosphate and urea).

| Models A/B | $SSE_A$ | $SSE_B$ | n | $P_A+1$ | $P_B+1$ | $AIC_A$ | $AIC_B$ | $AIC_{cA}$ | $AIC_{cB}$ | $\Delta$ | ProbB |
|---|---|---|---|---|---|---|---|---|---|---|---|
| $NCI_m/MI_m$ | 402.3 | 392.6 | 112 | 4 | 5 | 150.5 | 148.4 | 151.6 | 151.1 | -0.54 | 0.57 |

*Legend: A/B - Comparison of the two models (A and B) where A is the model with fewer parameters; PA and PB-Number of models A and B parameters respectively; AICc - AIC corrected; $\Delta$ - difference between the AICc values of the two compared models; Prob.B - Probability of B being correct. The index m from NCIm/MIM indicates that they are modified equations from the MI equation, as explained in the text.*

The present invention provides a method for the enzymatic kinetics study when two inhibitors are present, in which Michaelis-Menten integrated equations have been developed for the execution of kinetic analysis.

The here proposed method is useful for discrimination inhibition mechanisms and can be automated and implemented in real time.

Present description isn't naturally restricted in any way to the embodiments presented in this document, and a skilled person with middle knowledge in the field can foresee many possibilities for changing it, without parting from the general idea as defined in the claims. The above described preferential embodiments are obviously, combinable with each other. Additionally, the following claims define preferential embodiments.

## References

Akaike, H. "A new look at the statistical model identification", IEEE Trans. Automat .Contr. 19, 716-723, 1974.

Bezerra, R.M.F.; Fraga, I. and Dias, A. Albino. "Utilization of integrated Michaelis-Menten equations for enzyme inhibition diagnosis and determination of kinetic constants using Solver supplement of Microsoft Office Excel" Comput. Methods Programs Biomed. 109, pp. 26-31, 2013.

## Claims

1. A method for the determination of the enzymatic inhibition kinetics in simultaneous presence of two inhibitors, where one of the inhibitors is reaction product, which comprises the following steps:

   - reacting a substrate and an enzyme in the presence of two inhibitors, where one inhibitor is the reaction product;
   - monitoring concentrations of formed product, of remaining substrate and of inhibitor 1, the reaction product, at different time intervals of reaction time, through a methodology, adequate to determine the substrate and the product;
   - determining reaction time needed to obtain a determined concentration of reaction product through the integrated Michaelis-Menten equation of Table 2;
   - determining the difference between reaction real time, and the time obtained by the integrated Michaelis-Menten equation of Table 2;
   - calculating the SSE values using the values determined in the previous step;
   - performing a non-linear regression analysis to determine the values of the kinetic parameters that minimizes the SSE values for each inhibition model;
   - selecting adequate inhibition model, by comparing minimized value SSE and Akaike's information criterion; and
   - determining kinetic parameters for selected inhibition model, through the application of Michaels-Menten integrated equation corresponding to selected model.

**2.** Method according to preceding claim, wherein the adequate methodology to determine the substrate and the reaction product, comprises the reading of absorbency, mass spectrography, polarography and refraction index.

**3.** Method according to preceding claims, wherein the kinetic parameters determined for the selected inhibition model comprise: Km, Kic1, Kiu1, Kic2, Kiu2 and Vmax.

**Amended claims under Art. 19.1 PCT**

**1.** A method for the determination of the enzymatic inhibition kinetics in simultaneous presence of two inhibitors, where one of the inhibitors is reaction product, which comprises the following steps:

- reacting a substrate and an enzyme in the presence of two inhibitors, where one inhibitor is the reaction product;
- monitoring concentrations of formed product, of remaining substrate and of inhibitor 1, the reaction product, at different time intervals of reaction time, through a methodology, adequate to determine the substrate and the product;
- determining reaction time needed to obtain a determined concentration of reaction product through the integrated Michaelis-Menten equation of Table 2;
- determining the difference between reaction real time, and the time obtained by the integrated Michaelis-Menten equation of Table 2;
- calculating the SSE values using the values determined in the previous step;
- performing a non-linear regression analysis to determine the values of the kinetic parameters that minimizes the SSE values for each inhibition model;
- selecting adequate inhibition model, by comparing minimized value SSE and Akaike's information criterion; and
- determining kinetic parameters for selected inhibition model, through the application of Michaels-Menten integrated equation corresponding to selected model.

**2.** Method according to claim 1, wherein the adequate methodology to determine the substrate and the reaction product, comprises the reading of absorbency, mass spectrophotometry, polarography and refraction index.

**3.** Method according to previous claims, wherein the type of inhibition and the inhibition constant of each inhibitor are determined, in a situation where the inhibitor is a product of the reaction and no inhibitor is added to the reaction medium.

**4.** Method according to previous claims, wherein the type of inhibition and the inhibition constant of each inhibitor are determined, in a situation where two inhibitors are simultaneously present in the reaction.

**5.** Method according to claim 4, wherein each inhibitor is studied separately by eliminating the mutual interaction in the discrimination of the type of inhibition.

**6.** Method according to previous claims, wherein the kinetic parameters determined for the selected inhibition model comprise: Km, Kic1, Kiu1, Kic2, Kiu2 and Vmax.

**7.** Method according to the previous 6, wherein Km is the Michaelis constant, Kic1 and Kic2 are the dissociation constants of the enzyme-inhibitor complex of the inhibitor 1 and 2 respectively, Kiu1 and Kiu2 are dissociation constants of the complex enzyme-substrate-inhibitor of the inhibitor 1 and 2 respectively, and Vmax is the inhibition maximal rate.

## INTERNATIONAL SEARCH REPORT

| International application No |
| --- |
| PCT/IB2016/054324 |

**A. CLASSIFICATION OF SUBJECT MATTER**
INV.  G06F19/00
ADD.

According to International Patent Classification (IPC) or to both national classification and IPC

**B. FIELDS SEARCHED**

Minimum documentation searched (classification system followed by classification symbols)
G06F

Documentation searched other than minimum documentation to the extent that such documents are included in the fields searched

Electronic data base consulted during the international search (name of data base and, where practicable, search terms used)

EPO-Internal, WPI Data

**C. DOCUMENTS CONSIDERED TO BE RELEVANT**

| Category* | Citation of document, with indication, where appropriate, of the relevant passages | Relevant to claim No. |
| --- | --- | --- |
| X | BEZERRA R. M. F. ET AL: "Enzymatic Kinetic of Cellulose Hydrolysis Inhibition by Ethanol and Cellobiose", APPLIED BIOCHEMISTRY AND BIOTECHNOLOGY, vol. 126, no. 1, 1 July 2005 (2005-07-01), pages 49-59, XP055329759, DOI: 10.1007/s12010-005-0005-5 the whole document<br><br>-----<br><br>-/-- | 1-3 |

| [X] Further documents are listed in the continuation of Box C. | | [ ] See patent family annex. |
| --- | --- | --- |

\* Special categories of cited documents :

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 16 December 2016 | 22/12/2016 |

| Name and mailing address of the ISA/<br>European Patent Office, P.B. 5818 Patentlaan 2<br>NL - 2280 HV Rijswijk<br>Tel. (+31-70) 340-2040,<br>Fax: (+31-70) 340-3016 | Authorized officer<br><br>Godzina, Przemyslaw |
| --- | --- |

Form PCT/ISA/210 (second sheet) (April 2005)

## INTERNATIONAL SEARCH REPORT

International application No

PCT/IB2016/054324

C(Continuation). DOCUMENTS CONSIDERED TO BE RELEVANT

| Category* | Citation of document, with indication, where appropriate, of the relevant passages | Relevant to claim No. |
|---|---|---|
| X | SOUSA JR. R. ET AL: "Recent trends in the modeling of cellulose hydrolysis", BRAZILIAN JOURNAL OF CHEMICAL ENGINEERING, vol. 28, no. 4, 1 October 2011 (2011-10-01), pages 545-564, XP055329754, BR ISSN: 0104-6632, DOI: 10.1590/S0104-66322011000400001 the whole document | 1-3 |
| A | BEZERRA R. M. F. ET AL: "Utilization of integrated Michaelis-Menten equations for enzyme inhibition diagnosis and determination of kinetic constants using Solver supplement of Microsoft Office Excel", COMPUTER METHODS AND PROGRAMS IN BIOMEDICINE, vol. 109, no. 1, 26 September 2012 (2012-09-26), pages 26-31, XP055329828, NL ISSN: 0169-2607, DOI: 10.1016/j.cmpb.2012.08.017 cited in the application the whole document | 1-3 |
| A | CHOU T-C. ET AL: "A Simple Generalized Equation for the Analysis of Multiple Inhibitions of Michaelis-Menten Kinetic Systems*", THE JOURNAL OF BIOLOGICAL CHEMISTRY, vol. 252, no. 18, 25 September 1977 (1977-09-25), pages 6438-6442, XP055329764, the whole document | 1-3 |

Form PCT/ISA/210 (continuation of second sheet) (April 2005)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 03106698 A1

**Non-patent literature cited in the description**

• **AKAIKE, H.** A new look at the statistical model identification. *IEEE Trans. Automat .Contr.,* 1974, vol. 19, 716-723

• **BEZERRA, R.M.F. ; FRAGA, I. ; DIAS, A. ALBINO.** Utilization of integrated Michaelis-Menten equations for enzyme inhibition diagnosis and determination of kinetic constants using Solver supplement of Microsoft Office Excel. *Comput. Methods Programs Biomed.,* 2013, vol. 109, 26-31